(19) Europäisches Patentamt
European Patent Office
Office européen des brevets

(11) Publication number: **0 090 510**
**B1**

(12) **EUROPEAN PATENT SPECIFICATION**

(45) Date of publication of patent specification: **11.11.87**

(51) Int. Cl.⁴: **C 07 C 125/067,** A 61 K 31/27

(21) Application number: **83301174.5**

(22) Date of filing: **04.03.83**

(54) **O-carbamoyl salicylates and their preparation.**

(43) Date of publication of application:
**05.10.83 Bulletin 83/40**

(45) Publication of the grant of the patent:
**11.11.87 Bulletin 87/46**

(84) Designated Contracting States:
**CH DE FR GB IT LI**

(56) References cited:
**DE-A-2 755 224**
**FR-A-2 341 318**

**Chemical Abstracts vol. 76, no. 9, 28 February 1972, Columbus, Ohio, USA M. HEDAYATULLAH et al. "Polyhalo and polynitroaryl cyanates and iminocarbonates", page 350, column 2, abstract no. 45863u & Bull. Soc. Chim. Fr., no. 10, 1971, pages 3567-3**

**CHEMISCHE BERICHTE, vol. 99, no. 1 1966, VERLAG CHEMIE, Weinheim GRIGAT et al. "Umsetzung von Cyansäureestern mit Sulfonsäuren und Sulfonsäurederivaten bzw. mit Pseudohalogenwasserstoffen", pages 958ff**

**The file contains technical information submitted after the application was filed and not included in this specification**

(73) Proprietor: **Council of Scientific and Industrial Research**
**Rafi Marg**
**New Delhi 110 001 (IN)**

(72) Inventor: **Kamal, Ahmed**
**H. No. 8-2-619 "Sultan Manzil" Road No. 11**
**Nanjara Hills Hyderabad - 500034 (IN)**
Inventor: **Sattur, Pralhad Balvant Rao**
**H. No. 3-4-101 3/5 Rajendra Colony**
**Barkstpura Hyderabad - 500027 (IN)**
Inventor: **Thyagarajan, Gopalakrishna**
**Director's Bungalow Regional Research Lab.**
**Hyderabad - 500009 (IN)**

(74) Representative: **Warren, Keith Stanley et al**
**BARON & WARREN 18 South End Kensington**
**London W8 5BU (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 090 510 B1

## Description

This invention relates to O-carbamoyl salicylates, which are useful as analgesics, antipyretics and anti-inflammatory agents in mammals.

The most readily available and commonly used agents for relief of mild pain are mild analgesics like salicylates and derivatives of salicylic acid which have been used since 1838. The most important derivative of salicylic acid, acetylsalicylic acid (aspirin), was found pharmacologically useful only in 1899. The only other salicylate known is methyl salicylate used for topical application for relief in muscular and joint pains. Use of aspirin, particularly in high doses, produces side effects like gastrointestinal disorders.

In view of the severe side effects of aspirin, it was considered of interest to modify its chemical structure to overcome the side effects while retaining its beneficial analgesic action.

Accordingly, we turned our attention, amongst others, to O-carbamoyl salicylates, which have been found by us to meet the requirements.

Preparation of O-carbamoyl methyl salicylate has been reported by Ernst Grigat and Rlf Putter in chemische Bericht *99* (3), 958 (1966) who obtained same by the reaction of O-cyanatobenzoic acid methyl ester in benzene with p-toluene sulfonic acid. This report contains no hint of the possible pharmacological properties of the compounds. We have now discovered that those compounds possess analgesic, antipyretic and anti-inflammatory activity in mammals and hence may find use as drugs in the treatment of pain and inflammation and also as possible alternate to the widely employed drug, acetyl salicylic acid (aspirin).

In one aspect this invention provides O-carbamoyl salicylates represented by the general formula I

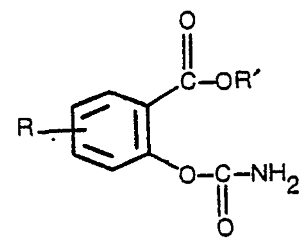

(I)

Wherein R represents H, halogens such as chloro, dichloro, bromo and iodo; alkyl or alkoxy group comprising of one to six carbon atoms or a nitro group, in various positions, and R′ represents an alkyl or chloro substituted alkyl group, straight or branched, comprising of two to six carbon atoms or an aromatic ring containing one or more substituents such as halogen, alkyl group, either straight or branched, comprising up to fifteen carbon atoms alkoxy group comprising of one to six carbon atoms and nitro group, for use as analgesic, antipyretic and/or anti-inflammatory agents.

The compounds of formula (I) wherein R represents H or an alkyl, alkoxy or nitro group are novel compounds.

In another aspect this invention provides a process for the preparation of O-carbamoyl salicylates of the general formula Ia,

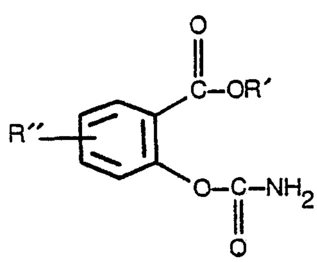

(Ia)

wherein R represents H, an alkyl or alkoxy group comprising one to six carbon atoms or a nitro group, in various positions, and R′ represents an alkyl or chloro substituted alkyl group, straight or branched, comprising one to six carbon atoms or an aromatic ring containing one or more substituents such as halogen, alkyl group, either straight or branched, comprising up to fifteen carbon atoms, alkoxy group comprising of one to six carbon atoms and nitro group, which comprises treating a compound of formula II

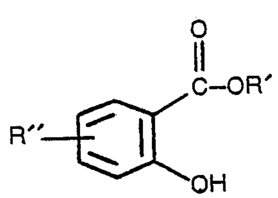

(II)

in which R'' and R' have the same meaning as before with chlorosulfonyl isocyanate represented by the formula

$$Cl.SO_2.NCO$$

The above reaction is carried out in the presence of aprotic solvents like aromatic hydrocarbons such as benzene, toluene and xylene, ethers such as diethyl ether, diisopropyl ether, glycol dimethyl ether, diethylene glycol, dimethyl ether, chloro hydrocarbons such as methylene chloride, propylene chloride, chloroform, carbon tetrachloride or trichloro ethylene, or esters such as ethyl acetate, butyl acetate propionic acid methyl ester, at temperatures ranging from −5°C to +60°C. The reaction is carried out for a period ranging from 60 minutes to 300 minutes. The compounds so produced are isolated from the reaction mixture by treatment with ice and could be crystallized from organic solvents like ethanol.

The compounds of the invention obtained in the present invention are characterized and identified by chemical and spectroscopic methods.

The following are some typical examples which illustrate the process of this invention.

## Example 1

O-Carbamoyl ethyl salicylate

In a four necked flask fitted with a mechanical stirrer, thermometer, condenser and dropping funnel was taken ethyl salicylate (7.8 gms) in dry methylene chloride (30 ml) Chlorosulfonyl isocyanate (4 ml) in methylene chloride (10 ml) was added dropwise over a period of 15 minutes with stirring and maintaining the temperature 5—10°C. On completion of the addition the reaction mixture was stirred and allowed to warm to ambient temperature (28°C). The stirring was further continued for another 30 minutes. After removal of methylene chloride under vacuum, the residue obtained, was added to crushed ice (50 gms) and left for 5 hours. The crude product obtained is filtered and recrystallized from ethanol. m.p. 150—152°C. Yield: 93%.

## Example 2

O-Carbamoyl phenyl salicylate

In a four necked flask fitted with a mechanical stirrer, thermometer, condenser and dropping funnel was taken phenyl salicylate (9.85 gms) in dry benzene (40 ml). Chlorosulfonyl isocyanate (4 ml) in benzene (8 ml) was added dropwise over a period of 12 minutes with stirring and maintaining the temperature of 25—30°C. After completion of addition, the reaction mixture was stirred for another 45 minutes. The reaction mixture was then added to crushed ice (100 gms) and stirred for 6 hours. Benzene was separated and the solid obtained in the aqueous part was filtered, dried and recrystallized from methanol. m.p. 141°C. Yield: 95%.

## Example 3

O-Carbamoyl-(3-pentadecylphenyl) salicylate

In a four necked flask fitted with a mechanical stirrer, thermometer, condenser and dropping funnel was taken 3-pentadecylphenyl salicylate (9.73 gms) in dry diethyl ether (50 ml). Chlorosulfonyl isocyanate (2 ml) in diethyl ether (6 ml) was added dropwise over a period of 20 minutes with stirring and maintaining the temperature 10—15°C. Once the addition is completed the reaction mixture was stirred and allowed to warm to ambient temperature (23°C). Diethyl ether was removed under vacuum. To the residue obtained, was added crushed ice (40 gms) and left for 3 hours. The crude product obtained was filtered and recrystallized from dilute ethanol (80%). m.p. 108—110°C. Yield: 87%.

Employing the procedure as described above in examples 1, 2 and 3, the following additional compounds, which illustrate the invention, were prepared.

O-carbamoyl methyl salicylate (m.p. 149—150°C)
O-carbamoyl-5-chloromethyl salicylate (m.p. 151—152°C)
O-carbamoyl-5-chloroethyl salicylate (m.p. 143°C)
O-carbamoyl propyl salicylate (m.p. 146—148°C)
O-carbamoyl-5-chloro propyl salicylate (m.p. 140°C)
O-carbamoyl isopropyl salicylate (m.p. 145—147°C)
O-carbamoyl-5-chloro isopropyl salicylate (m.p. 148—149°C)
O-carbamoyl butyl salicylate (m.p. 152—154°C)
O-carbamoyl isobutyl salicylate (m.p. 148—149°C)
O-carbamoyl cyclohexylsalicylate (m.p. 154—155°C)
O-carbamoyl-5-chloro-phenyl salicylate (m.p. 128—130°C)
O-carbamoyl-(2-methyl-phenyl) salicylate (m.p. 90—92°C)
O-carbamoyl-5-chloro(2-methyl-phenyl) salicylate (m.p. 94—96°C)
O-carbamoyl (3-methylphenyl) salicylate (m.p. 95—97°C)
O-carbamoyl (4-methylphenyl) salicylate (m.p. 98—99°C)
O-carbamoyl (2-chlorophenyl) salicylate (m.p. 155—157°C)
O-carbamoyl-5-chloro (2-chlorophenyl) salicylate (m.p. 158—159°C)

O-carbamoyl (4-chlorophenyl) salicylate (m.p. 164—165°C)
O-carbamoyl-5-chloro (4-chlorophenyl) salicylate (m.p. 158—159°C)
O-carbamoyl-(2,4-dichlorophenyl) salicylate (m.p. 158—159°C)
O-carbamoyl-5-chloro(2,4-dichlorophenyl) salicylate (m.p. 164—165°C)
O-carbamoyl (2,3-dichlorophenyl) salicylate (m.p. 160—162°C)
O-carbamoyl-5-chloro(2,5-dichlorophenyl) salicylate (m.p. 163—164°C)
O-carbamoyl (3-methyl-4-chlorophenyl) salicylate (m.p. 150—151°C)
O-carbamoyl-5-chloro(3-methyl-4-chlorophenyl) salicylate (m.p. 153°C)
O-carbamoyl-5-chloro(3-pentadecylphenyl) salicylate (m.p. 109—111°C)
O-carbamoyl (4-methoxyphenyl) salicylate (m.p. 120°C)
O-carbamoyl (4-ethoxyphenyl) salicylate (m.p. 124—126°C)
O-carbamoyl (2-nitrophenyl) salicylate (m.p. 163°C)
O-carbamoyl (4-nitrophenyl) salicylate (m.p. 165°C)

Table I gives analgesic and anti-inflammatory profile of some representative compounds of this invention.

TABLE I

Analgesic and Anti-Inflammatory Profile of Some Representative Compounds

Dose: 200 mg/kg        $LD_{50}$ i.p. mice 800 mg

Gross observations: No obvious gross effects pertaining to Central Nervous System or Autonomic Nervous System

| Sl. No. | Compound | Analgesic protection | Anti-inflammatory | % Inhibition |
|---|---|---|---|---|
| | | Writhing | Raw edema | Granuloma |
| 1 | O-carbamoyl methyl salicylate | 69.78 | 76.23 | 46.32 |
| 2 | O-carbamoyl ethylsalicylate | 95.00 | 34.86 | 28.63 |
| 3 | O-carbamoyl-5-chloro ethylsalicylate | 67.00 | 34.85 | 19.79 |
| 4 | O-carbamoyl phenylsalicylate | 72.00 | 93.19 | 57.65 |
| 5 | O-carbamoyl (2-chlorophenyl) salicylate | 70.00 | 35.00 | 35.06 |
| 6 | O-carbamoyl (4-chlorophenyl) salicylate | 83.00 | 37.88 | 41.45 |
| 7 | O-carbamoyl (2,4-dichlorophenyl) salicylate | 76.00 | 28.77 | 34.40 |
| 8 | O-carbamoyl (3-methylphenyl) salicylate | 84.00 | 48.50 | 33.25 |
| 9 | O-carbamoyl (3-methyl-4-chlorophenyl) salicylate | 68.00 | 28.90 | 24.04 |
| 10 | O-carbamoyl (3-pentadecylphenyl) salicylate | 45.00 | 12.10 | 33.55 |

**Claims**

1. O-carbamoyl salicylates represented by the general formula I

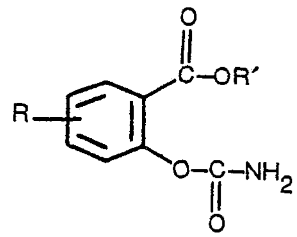

(I)

wherein R represents H, halogens such as chloro, dichloro, bromo and iodo; an alkyl or alkoxy group comprising one to six carbon atoms or a nitro group, in various positions, and R' represents an alkyl or chloro substituted alkyl group, straight or branched, comprising two to six carbon atoms or an aromatic ring containing one or more substituents such as halogen, an alkyl group, straight or branched, comprising up to fifteen carbon atoms, an alkoxy group comprising one to six carbon atoms or a nitro group, for use as analgesic, antipyretic and/or anti-inflammatory agents.

2. O-carbamoyl salicylates of the general formula Ia

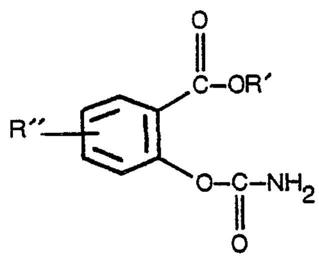

(Ia)

wherein R'' represents H, an alkyl or alkoxy group comprising one to six carbon atoms or a nitro group, and R' is as defined in claim 1.

3. A process for producing an O-carbamoyl salicylate having the general formula Ia defined in claim 2, which comprises treating a compound of formula II

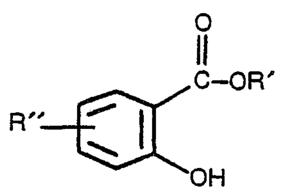

(II)

in which R'' and R' are as defined in claim 2, with chlorosulfonyl isocyanate represented by formula $Cl.SO_2NCO$, and isolating the resulting O-carbamoyl salicylate from the reaction mixture by treatment with ice.

4. A process as claimed in claim 3, wherein the reaction is carried out −5°C to 60°C for a period of 60 to 300 minutes.

5. A process as claimed in claim 3 or claim 4, wherein the solvent used is an aromatic hydrocarbon such as benzene, toluene or xylene, ether such as diethyl, diisopropyl ether, glycol dimethyl ether, diethylene glycol or dimethyl ether.

6. A process as claimed in any of claims 3 to 5, wherein the solvents used are chlorohydrocarbons such as methylene chloride, propylene chloride, chloroform, carbon tetrachloride or trichloro ethylene, tetrachloroethylene or esters such as ethyl acetate, butyl acetate or propionic acid methyl ester.

7. A process as claimed in any of claims 3 to 6, wherein the product crystallized from an organic solvent, preferably ethanol.

**Patentansprüche**

1. O-Carbamoylsalicylate, dargestellt durch die Formel I

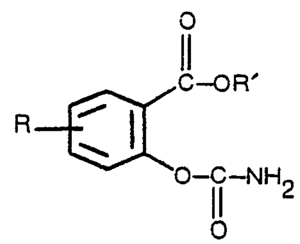

(I)

worin R H, Halogene wie Chlor, Dichlor, Brom und Jod, eine Alkylgruppe oder Alkoxygruppe, die eins bis sechs Kohlenstoffatome umfaßt, oder eine Nitrogruppe in verschiedenen Stellungen darstellt und R' eine geradkettige oder verzweigte Alkylgruppe oder mit Chlor substituierte Alkylgruppe, die zwei bis sechs Kohlenstoffatome umfaßt, oder einen aromatischen Ring, der einen oder mehrere Substituenten wie Halogen umfaßt, eine geradkettige oder verzweigte Alkylgruppe, die bis zu 15 Kohlenstoffatomen umfaßt, eine Alkoxygruppe, die eins bis sechs Kohlenstoffatome umfaßt, oder eine Nitrogruppe darstellt, zur Verwendung als analgetisches, antipyretisches und/oder entzündungshemmendes Mittel.

2. O-Carbamoylsalicylate der allgemeinen Formel Ia

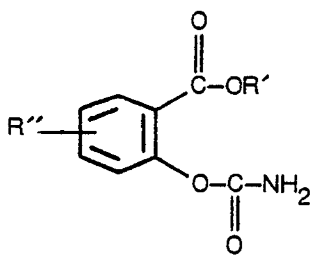

(Ia)

worin R'' H, eine Alkylgruppe oder Alkoxygruppe mit eins bis sechs Kohlenstoffatomen oder eine Nitrogruppe darstellt und R' wie in Anspruch 1 definiert ist.

3. Verfahren zur Herstellung eines O-Carbamoylsalicylates mit der in Anspruch 2 definierten allgemeinen Formel Ia, welches die Behandlung der Verbindung der Formel II,

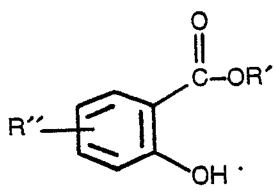

(II)

worin R'' und R' wie in Anspruch 2 definiert sind, mit Chlorsulfonylisocyanat, dargestellt durch die Formel $Cl.SO_2NCO$ und die Abtrennung des resultierenden O-Carbamoylsalicylates aus der Reaktionsmischung durch Behandlung mit Eis umfaßt.

4. Verfahren nach Anspruch 3, worin die Reaktion während eines Zeitraumes von 60 bis 300 min bei −5°C bis 60°C durchgeführt wird.

5. Verfahren nach Anspruch 3 oder 4, worin das verwendete Lösungsmittel ein aromatischer Kohlenwasserstoff, wie Benzol, Toluol oder Xylol, ein Ether wie Diethylether, Diisopropylether, Glycoldimethylether, Diethylenglycol oder Dimethylether ist.

6. Verfahren nach einem der Ansprüche 3 bis 5, worin die verwendeten Lösungsmittel Chlorkohlenwasserstoffe wie Methylenchlorid, Propylenchlorid, Chloroform, Kohlenstofftetrachlorid oder Trichlorethylen, Tetrachlorethylen oder Ester wie Ethylacetat, Butylacetat oder Propionsäuremethylester sind.

7. Verfahren nach einem der Ansprüche 3 bis 6, worin das Produkt aus einem organischen Lösungsmittel, vorzugsweise Ethanol, kristallisiert wird.

**Revendications**

1. O-carbamoylsalicylates représentés par la formule générale I

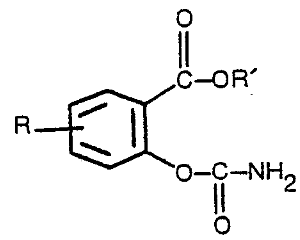

(I)

caractérisés ce que R représenté H, des halogènes tels que chloro, dichloro, bromo et iodo; un groupe alkyle ou alcoxyle comprenant un à six atomes de carbone ou un groupe nitro, en diverses positions, et R′ représente un groupe alkyle ou alkyle substitué par le chlore, droit ou ramifié, comprenant deux à six atomes de carbone ou un noyau aromatique contenant un ou plusieurs substituants tels qu'un halogène, un groupe alkyle droit ou ramifié comprenant jusqu'à quinze atomes de carbone, un groupe alcoxyle comprenant un à six atomes de carbone ou un groupe nitro, pour utilisation comme analgésiques, antipyrétiques et/ou antiinflammatoires.

2. O-carbamoylsalicylates de la formule générale Ia

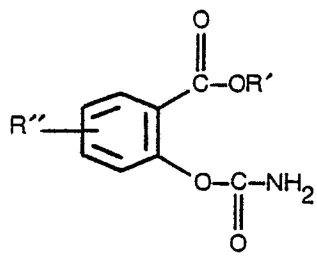

(Ia)

caractérisés en ce que R″ représente H, un groupe alkyle ou alcoxyle comprenant un à six atomes de carbone ou un groupe nitro et R′ est comme défini dans la revendication 1.

3. Procédé pour préparer un O-carbamoylsalicylate ayant la formule générale Ia définie dans la revendication 2, caractérisé en ce qu'il consiste à traiter un composé de formule II

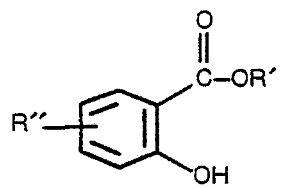

(II)

dans laquelle R″ et R′ sont comme définis à la revendication 2, par l'isocyanate de chlorosulfonyle représenté par la formule $Cl.SO_2NCO$ et à isoler du mélange réactionnel l'O-carbamoylsalicylate obtenu par traitement à la glace.

4. Procédé selon la revendication 3, caractérisé en ce que l'on conduit la réaction à −5 à 60°C pendant un temps de 60 à 300 minutes.

5. Procédé selon l'une des revendications 3 et 4, caractérisé en ce que le solvant utilisé est un hydrocarbure aromatique tel que le benzène, le toluène ou le xylène, un éther tel que l'éther diéthylique, l'éther diisopropylique, l'éther diméthylique du glycol, le diéthylèneglycol ou l'éther diméthylique.

6. Procédé selon l'une quelconque des revendications 3 à 5, caractérisé en ce que les solvants utilisés sont des chlorohydrocarbures tels que le chlorure de méthylène, le chlorure de propylène, le chloroforme, le tétrachlorure de carbone ou le trichloréthylène, le tétrachloréthylène ou des esters tels que l'acétate d'éthyle, l'acétate de butyle ou le propionate de méthyle.

7. Procédé selon l'une quelconque des revendications 3 à 6, caractérisé en ce que l'on cristallise le produit dans un solvant organique, de préférence l'éthanol.